# EUROPÄISCHE PATENTSCHRIFT

(11) **EP 1 304 298 B1**
(45) Veröffentlichungstag und Bekanntmachung des Hinweises auf die Patenterteilung: **03.08.2005**
(21) Anmeldenummer: 03000678.7
(22) Anmeldetag: 31.05.2000
(51) Int. Cl.: B65D 75/58

(54) **Packung zum Lagern von Sterilgut**
Package for a sterile product
Emballage pour le stockage d'un produit stérile

(30) Priorität: 12.07.1999 DE 19932458
(43) Veröffentlichungstag der Anmeldung: 23.04.2003
(62) Teilanmeldung aus: 00111674.8
(73) Patentinhaber: Lohmann & Rauscher GmbH, 2525 Schönau/Triesting (AT)
(72) Erfinder: Waldner, Wilhelm, Ing., 2525 Schönau/Triesting (AT); Leuprecht, Helmut, Dr., 1130 Wien (AT); Koch, Reinhard, 53489 Sinzig (DE)
(74) Vertreter: Patentanwälte Leinweber & Zimmermann

(56) Entgegenhaltungen:
- US-A- 4 337 862

## Beschreibung

Die Erfindung betrifft Innenverpackung für eine Packung zum Lagern und Darreichen von Sterilgut nach dem Oberbegriff des Patentanspruchs 1.

Derartige Packungen werden beispielsweise zum Bereitstellen von während einer Operation benötigten Verbandstoffen eingesetzt.

Dazu wird die Außenverpackung üblicherweise von einer unsterilen Person geöffnet. Aus der so geöffneten Außenverpackung entnimmt dann eine sterile Person die Innenverpackung, öffnet diese und entnimmt daraus das Sterilgut, wie etwa die benötigten Verbandstoffe.

Nach Abschluß einer Operation und vor Verschluß der Operationswunde muß durch eine Zählkontrolle sichergestellt werden, daß alle benutzten Verbandstoffe und sonstigen Sterilgüter aus dem Körper des Patienten entfernt worden sind. Zu diesem Zweck sind Packungen der eingangs beschriebenen Art üblicherweise mit zumindest den Inhalt der Packung angebenden Zählkarten ausgestattet. Nach den einschlägigen Vorschriften ist es erforderlich, daß die abschließende Zählkontrolle sowohl von einer sterilen Person im Operationssaal, als auch von einer unsterilen Person außerhalb des Operationssaals durchgeführt wird, bevor die Operationswunde verschlossen werden darf. Aus diesem Grund sind in jeder Packung der eingangs angegebenen Art mindestens zwei Zählkarten enthalten.

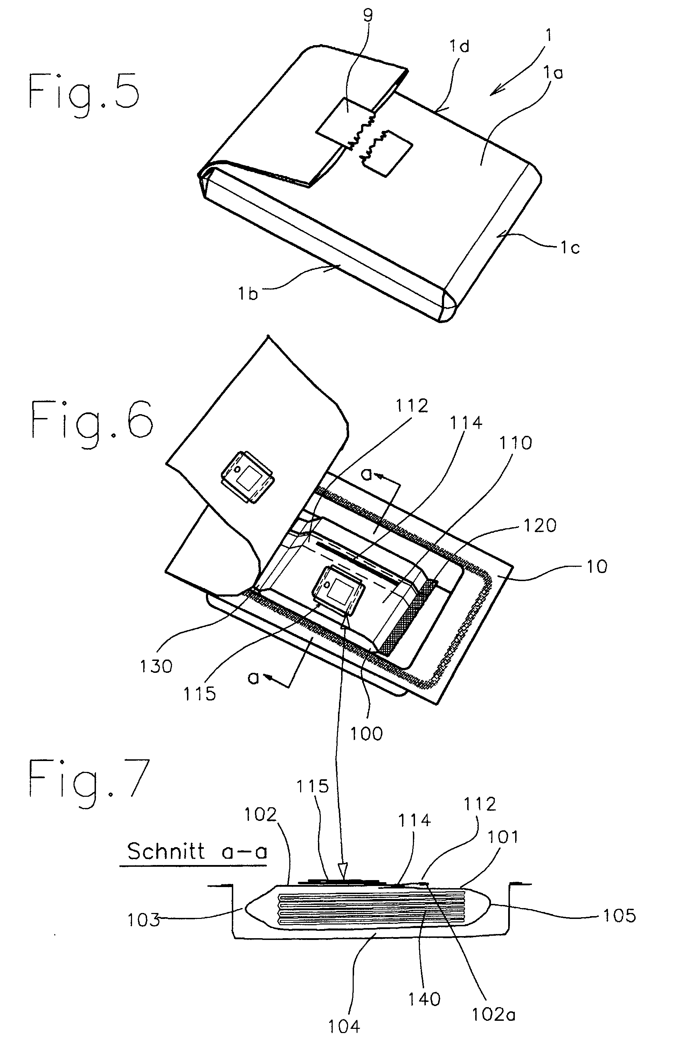

Eine mit diesen beiden Zählkarten ausgestattete Innenverpackung einer Packung der eingangs angegebenen Art nach dem Stand der Technik ist in Fig. 4 dargestellt. Bei dieser Packung ist auf der Außenseite einer in Form eines mit Klebestreifen verschlossenen Papierbeutels 1 verwirklichten und mit Verbandstoffprodukten befüllten Innenverpackung eine zweiteilige Zählkarte mit einem kreisförmigen Klebeelement 2 befestigt. Die Zählkarte selbst besteht aus einer zweiteiligen Klebeetikette 4, 5 mit einem den Inhalt der Innenverpackung darstellenden Aufdruck 8 und einem Trägerstreifen 3 aus nichtklebendem Silikonpapier, auf dem die Klebeetiketten 4, 5 angebracht sind. Die beiden Klebeetiketten 4, 5 und der Trägerstreifen 3 sind mit einer Perforation 7 versehen. Diese Perforation bildet eine Trennlinie, welche die Trennung der einzelnen Klebeetiketten voneinander ermöglicht. Weiter ist in Fig. 4 erkennbar, daß jede der Klebeetiketten 4, 5 mit einer Lochstanzung 6 versehen ist, die zum Aufhängen der gesammelten Zählkarten dient.

Zur Herstellung einer fertigen Packung wird die in Fig. 4 dargestellte Anordnung in eine Außenverpackung (in Fig. 4 nicht dargestellt) eingelegt und sterilisiert. Zur Bereitstellung der in der in Fig. 4 dargestellten Innenverpackung 1 enthaltenen Verbandstoffprodukte während einer Operation wird zunächst die in Fig. 4 nicht dargestellte Außenverpackung von einer unsterilen Person geöffnet. Danach entnimmt eine sterile Person, wie etwa eine OP-Schwester die Innenverpackung 1. Im weiteren Verlauf nimmt die sterile Person die zweiteilige Zählkarte von der Innenverpackung 1 ab, trennt die Zählkarten 4, 5 längs der Trennlinie 7 voneinander und übergibt eines der Zählkartenteile der unsterilen Person.

Dabei muß darauf geachtet werden, daß sowohl die sterile Person als auch die unsterile Person die zu übergebende Zählkarte jeweils an einem ihrer äußeren Enden anfaßt, um so eine Kontaminierung der sterilen Person zu vermeiden. Nicht verhindert werden kann dabei jedoch, daß die unsterile Person der sterilen Person während der Übergabe sehr nahe kommt. Daher ist die Übergabe der Zählkarte an die unsterile Person mit einer erheblichen Kontaminationsgefahr verbunden, die nur bei einer äußerst sorgfältigen Handhabung auf einem noch akzeptablen Maß gehalten werden kann. Ein weiteres Problem bei der Benutzung von Packungen der in Fig. 4 dargestellten Art ist darin zu sehen, daß beim Nachreichen von Verbandstoffen, was beispielsweise bei einem unerwarteten Operationsverlauf notwendig werden kann, der oben bereits erläuterte Vorgang des Öffnens der Außenverpackung und der Entnahme der Innenverpackung wiederholt werden muß, wobei die sterile Person jedoch bereits durch Blut des zu operierenden Patienten kontaminiert ist. Daher besteht hier die Gefahr, daß die unsterile Person bei der Übergabe der Zählkarte beschmutzt bzw. kontaminiert wird. Ferner steht der unsterilen Person nach Übergabe der Zählkarte nur eine kontaminierte bzw. blutige Zählkarte für die Kontrolle und Dokumentation zur Verfügung. Um das zu verhindern, bedarf es derzeit eines sehr aufwendigen Handlings.

Die Fig. 4 und 5 zeigen perspektivische Ansichten herkömmlicher Innenverpackungen für Packungen zum Lager von Sterilgut. Danach weist die in Form eines Papierbeutels 1 gebildete Innenverpackung eine erste im wesentlichen rechteckige und der Form des darin aufgenommenen Sterilgutes entsprechende Hauptfläche 1a auf, die über Randabschnitte 1b, 1c und 1d mit einer gegenüberliegenden Hauptfläche des Papierbeutels 1 verbunden ist. Der Papierbeutel ist nur einseitig längs des Randabschnittes 1c verschlossen. Längs seines parallel zu diesem Randabschnitt 1c verlaufenden Randes weist der Papierbeutel 1 eine Öffnung auf. Dieser Randabschnitt ist auf die Hauptfläche 1a zurückgefaltet und, wie vorstehend bereits erläutert, mit einem Klebestreifen 9 auf der Hauptfläche 1a befestigt, wodurch gleichzeitig ein sicherer Verschluß des Papierbeutels 1 erfolgt. Nach der eingangs bereits erläuterten Entnahme der Innenverpackung 1 aus einer Außenverpackung wird die Innenverpackung geöffnet und der Inhalt der Innenverpackung kontrolliert. Dazu wird der Klebestreifen 9 aufgerissen bzw. von der Hauptfläche 1a getrennt. Durch die dann freiwerdende Öffnung des Papierbeutels längs dessen parallel zu dem querverlaufenden Rand 1c verlaufenden Rand kann das Sterilgut kontrolliert bzw. aus dem Papierbeutel 1 entnommen werden.

Ferner sind Packungen für flüssige Lebensmittel, die zum Bilden einer Öffnung im Bereich einer Hauptfläche der Packung vorbeseitet sind, in der US-A-4337862 angegeben.

Es hat sich gezeigt, daß es im besonderen in der Hektik einer Operation bei der Entnahme des Sterilgutes aus den bekannten Innenverpackungen häufig zu einer Beschädigung bzw. zum Einreissen der die Innenverpackung bildenden Papierhülle kommt. Dadurch kann eine vorzeitige Kontaminierung des in der Innenverpackung aufgenommenen Sterilgutes erfolgen.

Angesichts dieser Probleme im Stand der Technik liegt dieser Erfindung die Aufgabe zugrunde, eine Innenverpackung für eine erfindungsgemäße Packung bereitzustellen, welche auch in der Hektik einer Operation eine rasche Öffnung und sichere Handhabung bei der Übergabe des Sterilgutes ermöglicht.

Erfindungsgemäß wird diese Aufgabe durch die im kennzeichnenden Teil des Patentanspruchs 1 angegebene Weiterbildung herkommlicher Innenverpackungen gelöst.

Diese Erfindung geht auf die Erkenntnis zurück, daß die im Stand der Technik auftretenden Probleme im wesentlichen darauf zurückzuführen sind, daß der Randbereich des Papierbeutels nur eine verhältnismäßig geringe Öffnungsfläche zur Verfügung stellt. Aus diesem Grund kann es beim Eingreifen in den Papierbeutel zur Entnahme des Sterilgutes daraus sehr leicht zu einer Beschädigung des Papierbeutels und einer vorzeitigen Kontaminierung des Sterilgutes kommen, wobei im besonderen die beim Aufreissen entstehenden und sich auf dem Sterilgut ablagernden Papierstäube zu Komplikationen, wie etwa Verkapselungen nach der Operation führen können.

Im Gegensatz dazu ist die Hülle der erfindungsgemäßen Innenverpackung zur Bildung einer Entnahmeöffnung im Bereich ihrer Hauptflächen vorbereitet. In diesem Bereich kann eine wesentlich größere Entnahmeöffnung gebildet werden. Dadurch wird selbst in der Hektik einer Operation eine Entnahme des Sterilgutes aus der Innenverpackung ermöglicht, ohne daß es zu Kontaminierungen mit Papierstäuben o. dgl. kommt.

Bei einer besonders bevorzugten Ausführungsform der Erfindung ist die Hülle zum Bilden einer sich ausgehend von der Hauptfläche bis über einen daran angrenzenden Rand des darin aufgenommenen Sterilgutes hinweg erstreckenden Entnahmeöffnung vorbereitet. Mit einer so vorbereiteten Innenverpackung wird eine besonders leichte Überprüfbarkeit des Inhaltes der Innenverpackung bzw. eine besonders einfache Stückzahlkontrolle durch Sicht auf die Falzkanten der in der Innenverpackung aufgenommenen Produkte, wie etwa Kompressen ermöglicht, weil die sich über den Rand des Sterilgutes erstreckende Entnahmeöffnung eine freie Sicht auf diese Falzkanten erlaubt. Im übrigen wird auf diese Weise auch noch ein besonders einfacher Zugriff auf das in der Innenverpackung aufgenommene Produkt ermöglicht. Dabei kann eine kontaminationsfreie Übergabe dieses Produktes erreicht werden, wenn die Hauptfläche auch nach Bildung der Entnahmeöffnung noch von einem Teil der Hülle abgedeckt wird. In diesem Fall kann das in der Innenverpackung aufgenommene Produkt durch die starre, nicht zum Öffnen der Innenverpackung benötigte Hand fixiert werden, in dem es über die noch daran anliegende Hülle in der Innenverpackung festgehalten wird, während der im Bereich der Entnahmeöffnung freiliegende Rand des Sterilgutes ohne Behinderung durch die das Sterilgut in der Innenverpackung haltende Person erfaßt werden kann.

Ähnlich wie bei der anhand der Fig. 5 erläuterten bekannten Innenverpackung weist auch die Hülle der erfindungsgemäßen Innenverpackung eine das Sterilgut zumindest teilweise umlaufende Materialbahn auf. Dabei weist diese Materialbahn der erfindungsgemäßen Innenverpackung einen ersten Abschnitt und einen zweiten Abschnitt auf, von denen jeder einen Teil der Hauptfläche bildet, wobei sich diese Abschnitte im Bereich der Hauptfläche teilweise überlappen. Dadurch wird einerseits eine vollständige Abdeckung des Sterilgutes durch die Materialbahn im Bereich der Hauptfläche erreicht, während andererseits eine besonders einfache Öffnung im Bereich der Hauptfläche erzeugt werden kann, in dem der erste Abschnitt und der zweite Abschnitt ausgehend von der überlappenden Anordnung voneinander getrennt werden.

Herstellungstechnisch hat es sich als besonders günstig erwiesen, wenn die erfindungsgemäße Innenverpackung in Form eines aus einer Materialbahn gebildeten Schlauchbeutels hergestellt ist, wobei die Materialbahn einen ersten, einen Teil der Hauptfläche bildenden Abschnitt, einen zweiten einen weiteren Teil der Hauptfläche bildenden Abschnitt, einen dritten eine erste Umlaufachse teilweise umlaufenden Abschnitt, einen vierten, die der Hauptfläche entgegengesetzte Begrenzungsfläche der Innenverpackung bildenden Abschnitt und einen fünften eine zweite vorzugsweise etwa parallel zu der ersten Umlaufachse verlaufende Umlaufachse teilweise umlaufenden Abschnitt aufweist, wobei der dritte Abschnitt einen zwischen dem ersten Abschnitt und dem vierten Abschnitt angeordneten Randbereich der Innenverpackung bildet und der fünfte Abschnitt einen weiteren zwischen dem vierten Abschnitt und dem zweiten Abschnitt angeordneten Randbereich der Innenverpackung bildet.

Bei dieser Anordnung kann die Hülle besonders einfach zum Bilden der Entnahmeöffnung im Bereich der Hauptfläche vorbereitet werden, wenn der erste Abschnitt und der zweite Abschnitt sich längs eines etwa parallel zu den Umlaufachsen bzw. in Längsrichtung des Schlauchbeutels verlaufenden Überlappungsbereiches überlappen, wobei diese Abschnitte die in Längsrichtung des Schlauchbeutels verlaufenden Ränder der Materialbahn bilden.

Zur Stabilisierung der erfindungsgemäßen Innenverpackung hat es sich als besonders günstig erwiesen, wenn der erste Abschnitt und der zweite Abschnitt in dem Überlappungsbereich lösbar miteinander verbunden, insbesondere verklebt oder versiegelt sind. Dabei kann sowohl eine Kaltsiegelung als auch eine Heißsiegelung zum Einsatz kommen. Zur Vermeidung einer Kontamination des in der Innenverpackung aufgenommenen Sterilgutes wird die Haltekraft der Verbindung zweckmäßigerweise so gering gewählt, daß beim Lösen der Verbindung keine Beschädigung des ersten Abschnittes oder des zweiten Abschnittes erfolgt.

Wenn der zweite Abschnitt auf seiner dem Sterilgut zugewandten Seite mit dem ersten Abschnitt verbunden ist und in seiner sich senkrecht zu den Umlaufachsen erstreckenden Richtung eine die Hälfte der Breite der Hauptfläche überschreitende Breite aufweist, kann die Entnahmeöffnung durch Abheben des zweiten Abschnittes von dem ersten Abschnitt gebildet werden, wobei der erste Abschnitt eine besonders große Fläche zum Anbringen eines beispielsweise in Form eines Klebeetiketts gebildeten Informationsträgers zur Verfügung stellt. Bei dieser Ausführungsform der Erfindung kann der Informationsträger also im Bereich des ersten Abschnittes auf der Hauptfläche der Innenverpackung angeordnet werden, ohne daß dadurch die Bildung der Entnahmeöffnung beeinträchtigt wird, während gleichzeitig eine besonders einfache Entfernung des Informationsträgers von der Innenverpackung ohne Kontaminierung des Sterilgutes möglich ist, weil der den Informationsträger tragende Abschnitt der Innenverpackung zunächst von dem ersten Abschnitt bzw. dem Sterilgut abgehoben werden kann und erst in diesem Zustand die Abnahme des Informationsträgers erfolgt. Durch diese Anordnung wird also eine dezentrale Positionierung des Informationsträgers ermöglicht, so daß dieser die Längsnaht des Schlauchbeutels nicht überlappt.

Hinsichtlich der Fixierung des in der geöffneten Innenverpackung aufgenommenen Sterilgutes mit der starren, nicht öffnenden Hand hat es sich als besonders günstig erwiesen, wenn der erste Abschnitt längs einer parallel zum Überlappungsbereich verlaufenden Linie auf sich selbst zurückgefaltet ist, so daß ein Haltebereich doppelter Materialstärke im Bereich der Hauptfläche bereitgestellt wird. Dabei kann ein besonders großer Haltebereich bereitgestellt werden, wenn auch der erste Abschnitt in einer sich senkrecht zu den Umlaufachsen verlaufenden Richtung eine die Hälfte der Breite der Hauptfläche überschreitende Breite aufweist.

Die Bereitstellung eines Haltebereichs doppelter Materialstärke wird bei der zuletzt beschriebenen Ausführungsform dadurch erreicht, daß der auf sich selbst zurückgefaltete Teil des ersten Abschnitts nicht vollständig von dem zweiten Abschnitt überlappt wird, so daß ein Rand des zweiten Abschnittes auch dann noch erfaßt werden kann, wenn das Sterilgut in der Innenverpackung im Bereich des auf sich selbst zurückgefalteten ersten Abschnittes erfaßt wird. Derartige Innenverpackungen sind besonders gut für verhältnismäßig große Produkte, wie etwa sterile Kompressen geeignet. Daneben kann eine erfindungsgemäße Innenverpackung aber auch für kleine Produkte oder nebeneinander darin angeordnete Produkte benutzt werden. Bei Einsatz einer erfindungsgemäßen Innenverpackung für besonders kleine Produkte ist es häufig nicht möglich, die Innenverpackung mit einer Hand zu halten und mit der anderen Hand zu öffnen, weil nicht genügend Griffraum für die haltende Hand zur Verfügung steht. In diesem Fall ist es besonders günstig, wenn die Innenverpackung mit beiden Händen geöffnet wird. Dazu kann der auf sich selbst zurückgefaltete Teil des ersten Abschnittes vollständig von dem zweiten Abschnitt überlappt werden. Die Öffnung der Innenverpackung erfolgt in diesem Fall so, daß die Innenverpackung auf einer Unterlage abgelegt wird, der auf sich selbst zurückgefaltete Teil des ersten Abschnittes zusammen mit dem diesen überlappenden Teil des zweiten Abschnittes hochgeklappt wird, dann die freiliegenden Ränder dieser Abschnitte mit jeweils einer Hand erfaßt und voneinander getrennt werden. Diese Handhabung ist auch dann von besonderem Vorteil, wenn in der Innenverpackung nebeneinander angeordnete Produkte aufgenommen sind, weil dann die ansonsten bestehende Gefahr beseitigt ist, daß das nicht festgehaltene sterile Gut während des Öffnungsvorgangs aus der Innenverpackung herausfällt.

Bei der Öffnung der erfindungsgemäßen Innenverpackung wird die Gefahr einer Beschädigung der im allgemeinen aus Papier bestehenden Hülle weiter verringert , weil ein freiliegender Rand des ersten Abschnitts und/oder des zweiten Abschnittes, also im allgemeinen die Längskante der Materialbahn zur Bildung einer verstärkten Anfaßlasche zumindest teilweise auf sich selbst zurückgefaltet ist.

Wie eingangs bereits erläutert wird die erfindungsgemäße Innenverpackung vorzugsweise in Form eines Schlauchbeutels verwirklicht. Dazu ist der erste Abschnitt und/oder der zweite Abschnitt längs mindestens eines quer zu den Umlaufachsen verlaufenden Verbindungsbereiche mit dem vierten Abschnitt verbunden, insbesondere verklebt oder versiegelt. Zweckmäßigerweise erfolgt die Verbindung längs beider senkrecht zu den Umlaufachsen verlaufenden Ränder des ersten Abschnittes und des zweiten Abschnittes. Zur Vermeidung einer die Erzeugung von Papierstaub verursachenden Beschädigung der Innenverpackung ist auch die Haltekraft dieser Verbindung vorzugsweise so gering, daß beim Lösen dieser Verbindung keine Beschädigung des ersten, zweiten oder vierten Abschnittes erfolgt.

Die Gefahr einer unbeabsichtigten Beschädigung der Innenverpackung kann weiter verringert werden, wenn sich die Verbindung des ersten Abschnittes mit dem zweiten Abschnitt nicht mit der Verbindung des ersten bzw. zweiten Abschnittes mit dem vierten Abschnitt kreuzt, weil ein Einreissen des Papiers oder Ablösen von Papierteilchen beim Öffnen der Innenverpackung vorzugsweise an derartigen Kreuzungspunkten auftritt. Zur Vermeidung derartiger Kreuzungspunkte wird besonders bevorzugt die Verbindung des ersten Abschnittes mit dem zweiten Aschnitt nicht über die gesamte Länge des Verbindungsbereiches ausgeführt, weil so gleichzeitig eine leichtere Handhabung beim Öffnen ermöglicht wird.

Nachstehend wird die Erfindung unter Bezugnahme auf die Zeichnung, auf die hinsichtlich aller erfindungswesentlichen und in der Beschreibung nicht näher herausgestellten Einzelheiten ausdrücklich verwiesen wird, erläutert. In der Zeichnung zeigt:
- Fig. 1: eine perspektivische Ansicht einer nicht zur Erfindung gehörenden, eine Innenvepackung zum Lagern von Sterilgut enthaltenden, Packung,
- Fig. 2: eine schematische Seitenansicht einer Vorrichtung zum Herstellen der in Fig. 1 dargestellten Packung,
- Fig. 3: eine schematische Draufsicht auf die in Fig. 2 dargestellte Vorrichtung,
- Fig. 4: eine perspektivische Ansicht einer Innenverpackung einer Packung zum Lagern von Sterilgut nach dem Stand der Technik,
- Fig. 5: eine perspektivische Ansicht einer herkömmlichen Innenverpackung,
- Fig. 6: eine perspektivische Ansicht einer in einer Außenverpackung aufgenommenen erfindungsgemäßen Innenverpackung,
- Fig. 7: eine Schnittansicht längs der in Fig. 6 angegebenen Schnittebene a-a,
- Fig. 8 und 9: Darstellungen zur Erläuterung der Handhabung der in den Fig. 6 und 7 gezeigten Innenverpackung,
- Fig. 10: eine Schnittdarstellung einer Innenverpackung gemäß einer weiteren Ausführungsform der Erfindung,
- Fig. 11: eine Schnittdarstellung einer zum Aufnehmen kleiner oder nebeneinander angeordneter Produkte geeigneten erfindungsgemäßen Innenverpackung und
- Fig. 12 und 13: Darstellungen zur Erläuterung der Handhabung der in Fig. 11 dargestellten Innenverpackung.

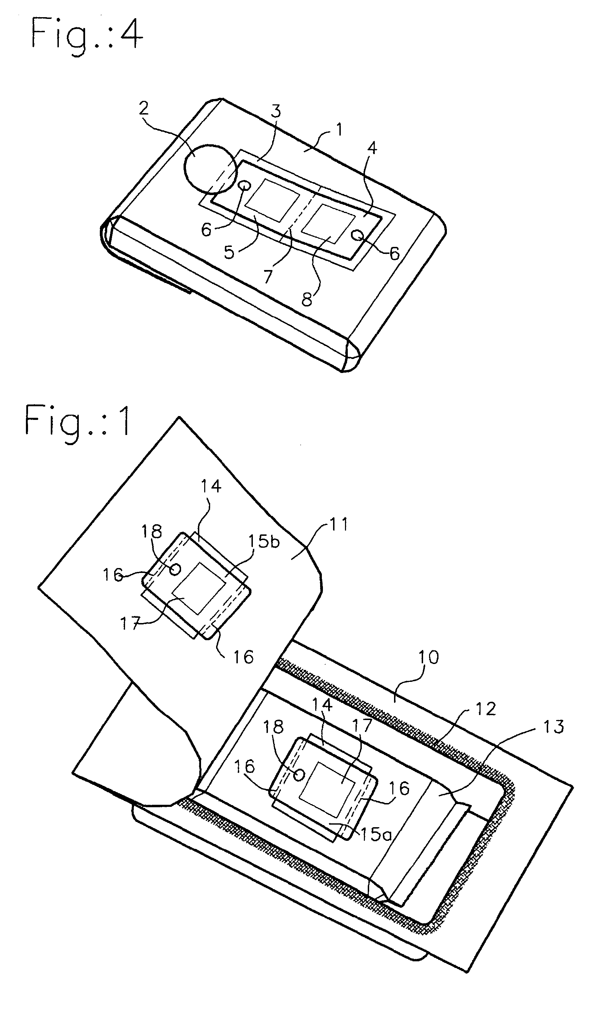

Die in Fig. 1 dargestellte Packung umfaßt eine Innenverpackung 13 in Form eines maschinell befüllten und verschlossenen Papierschlauchbeutels 13 sowie eine in Form einer tiefgezogenen Schale 10 mit einer aufgesiegelten Papierdeckbahn 11 gebildete Außenverpackung. Bei der Herstellung dieser Packungen wird der Papierschlauchbeutel 13 in einer Verpackungsmaschine in die tiefgezogene Schale 10 eingelegt. Vor dem Aufsiegeln der Papierdeckbahn 11 werden Zählkarten getrennt voneinander einerseits auf den Papierschlauchbeutel 13 und andererseits an der dem Innenraum der tiefgezogenen Schale 10 zugewandten Seite der Papierdeckbahn 11 angebracht.

Dabei umfaßt jede der Zählkarten ein mit einem Aufdruck 17 versehenes Klebeetikett 15a und 15b, deren dem Papierschlauchbeutel 13 bzw. der Innenseite der Papierdeckbahn 11 zugewandte Klebefläche teilweise von einer Trägerfolie 14 abgedeckt ist. Jede der Klebeetiketten 15a und 15b umfaßt jedoch parallel zueinander verlaufende und beidseits der nichtklebenden Trägerfolie angeordnete Klebeflächen, von denen jede mit einer parallel zu einem Rand der Trägerfolie 14 verlaufenden Perforation 16 versehen ist. Mit diesen Klebeflächen können die Klebeetiketten auf dem Papierschlauchbeutel 13 bzw. die Innenseite der Papierdeckbahn 11 aufgeklebt werden, während die Perforationen 16 ein einfaches, sicheres und beschädigungsfreies Abnehmen der Klebeetiketten 15a und 15b von dem Papierschlauchbeutel 13 bzw. der Papierdeckbahn 11 ermöglichen.

Wie in Fig. 1 weiter dargestellt, weist jede der Klebeetiketten bzw. Zählkarten 15a und 15b auch noch eine Lochstanzung 18 auf, die zum Aufhängen der gesammelten Zählkarten eingesetzt werden kann.

Beim Einsatz der in Fig. 1 dargestellten Packung wird zunächst die aus der tiefgezogenen Schale 10 und Papierdeckbahn 11 bestehende Außenverpackung durch eine unsterile Person geöffnet, in dem die Papierdeckbahn von der tiefgezogenen Schale abgezogen wird. Dieser Zustand ist in Fig. 1 dargestellt. Danach entnimmt eine sterile OP-Schwester den das Sterilgut, wie etwa Verbandstoffprodukte, enthaltenden Papierschlauchbeutel 13. Die OP-Schwester kann dann die Zählkarte bzw. das Klebeetikett von dem Papierschlauchbeutel 13 abnehmen und den Beutelinhalt überprüfen. Andererseits kann die auf der Innenseite der Papierdeckbahn 11 angebrachte Zählkarte 15b ohne Gefahr einer Kontamination des Papierschlauchbeutels 13 von der unsterilen Person von der Innenseite der Papierdeckbahn 11 abgenommen werden, die die Außenverpackung sowieso in den Händen hält. Dadurch wird eine Kontaminationsgefahr sicher ausgeschaltet.

Ein entscheidender Vorteil der gerade erläuterten Packung ist also darin zu sehen, daß aufgrund der getrennt angebrachten Zählkarten 15 und 15b die sterile und die unsterile Person nicht mehr, wie bei der alten Verpackungsweise mit den Händen nahe zusammenkommen müssen. Dadurch wird die Kontaminationsgefahr über die Zählkarten ausgeschaltet und eine Voraussetzung für eine kontaminationsfreie Dokumentation gewährleistet.

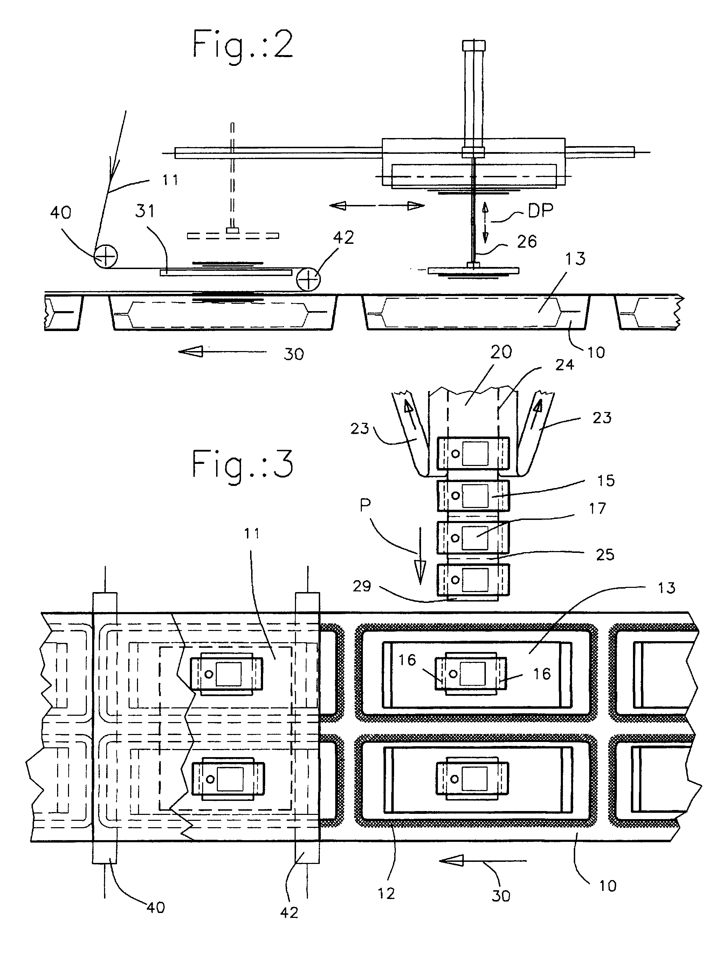

In den Fig. 2 und 3 ist die Anbringung der Zählkarten auf der in Fig. 1 dargestellten Packung vereinfacht dargestellt.

Dazu werden mit den benötigten Informationen, wie etwa dem Packungsinhalt, der Menge, dem Verfallsdatum, der Chargenbezeichnung, notwendigen Prüfzeichen u. dgl., bedruckte Klebeetiketten 15, die auf einer Trägerfolie 20 aus Silikonfolie oder einer anderen nichtklebenden Substanz angebracht sind, benutzt. Dabei sind die Klebeetiketten bereits in einer gewünschten Anordnung positioniert. Der aus der Trägerfolie und den Klebeetiketten bestehende Streifen wird dann getaktet durch eine Zuführeinrichtung in einer durch den Pfeil P bezeichneten Zuführrichtung zugeführt. Gleichzeitig werden während des Zuführvorgangs zwei parallel zur Zuführrichtung P verlaufende Randstreifen 23 der Trägerfolie 20 mit Hilfe einer bereits vorhandenen Perforation 24 abgetrennt. Die danach verbleibende Trägerfolie 29 ist in einer senkrecht zur Zuführrichtung P verlaufenden Richtung schmaler als die Klebeetiketten 15, so daß eine selbstklebende Unterseite der Klebeetiketten 15 beidseitig über die Trägerfolie 29 hinausragt. Die einzelnen Klebeetiketten 15 sind in der Zuführrichtung P voneinander beabstandet. Dadurch ergibt sich ein Überstand der Trägerfolie in der Zuführrichtung P.

Die aus der Trägerfolie 29 und den Klebeetiketten 15 bestehenden Zählkarten werden nun mittels einer in der Zeichnung nicht näher dargestellten Fördereinrichtung über den mit Hilfe einer ersten Fördereinrichtung in einer senkrecht zur Zuführrichtung P verlaufenden und durch den Pfeil 30 bezeichneten Richtung geförderten tiefgezogenen Folien 10 positioniert und durch einen Stempel 26 (vgl. Fig. 2) auf bereits in den tiefgezogenen Schalen 10 angeordneten Papierschlauchbeuteln befestigt. Dazu ist der Stempel 26, wie in Fig. 2 durch den Doppelpfeil DP angedeutet in einer senkrecht zur Förderrichtung der ersten Fördereinrichtung und der Zuführrichtung aufgespannten Ebene verlaufenden Richtung hin- und hergehend bewegbar. Im weiteren Verlauf der Förderung der tiefgezogenen Schalen 10 längs der durch den Pfeil 30 angegebenen vorgegebenen Bahn wird die Öffnung der einzelnen tiefgezogenen Schalen mit einer Papierdeckbahn 11 verschlossen.

Die Papierdeckbahn 11 wird mit einer eine erste Umlenkrolle 40 und eine zweite Umlenkrolle 42 aufweisenden Fördereinrichtung derart zugeführt, daß sie zwischen der ersten Umlenkrolle 40 und der zweiten Umlenkrolle 42 in einer der Förderrichtung 30 der ersten Fördereinrichtung entgegengestetzten Richtung gefördert wird und erst nach Umlaufen der zweiten Umlenkrolle 42 parallel zur Förderrichtung 30 der ersten Fördereinrichtung gefördert wird und auf die tiefgezogene Schale 10 aufgesiegelt werden kann.

In dem zwischen der ersten Umlenkrolle 40 und der zweiten Umlenkrolle 42 liegenden Streckenabschnitt werden die Klebeetiketten auf der im fertiggestellten Zustand dem Innenraum der tiefgezogenen Schale 10 zugewandten Innenseite der Papierdeckbahn 11 angebracht. Dazu ist in dem Zwischenraum zwischen der Papierdeckbahn 11 und den längs der vorgegebenen Bahn geförderten tiefgezogenen Schale im Bereich zwischen der ersten Umlenkrolle 40 und der zweiten Umlenkrolle 42 eine Gegenplatte 31 angebracht, mit der eine Beschädigung der Papierdeckbahn 11 während des Anbringens der Klebeetiketten darauf verhindert werden kann. Wie in Fig. 2 durch den Doppelpfeil 44 angedeutet, ist die zum Zuführen der Klebeetiketten ausgelegte Zuführeinrichtung und der Stempel 26 längs der vorgegebenen Bahn der tiefgezogenen Schalen 10 hin- und hergehend bewegbar. Dadurch wird erreicht, daß die in Fig. 3 dargestellte Befestigungseinrichtung in einer ersten in Fig. 2 mit durchgezogenen Linien dargestellten Position zum Anbringen von Klebeetiketten an den Papierschlauchbeuteln 13 und in einer zweiten in Fig. 2 strichliert dargestellten Position zum Anbringen der Klebeetiketten auf der Innenseite der Papierdeckbahn 11 betrieben werden kann.

Wie besonders deutlich der Fig. 3 zu entnehmen ist, sind die einzelnen Perforationen 16 beidseits, außerhalb und parallel zur Trägerfolie angeordnet. Dadurch kann die nur an den Rändern aufgeklebte Zählkarte leicht mit zwei Fingern gefaßt und von der Packung gelöst werden.

Weiter ist der Fig. 3 zu entnehmen, daß die Vorrichtung zur Fertigstellung von zwei oder mehr parallel zueinander geförderten Packungen ausgelegt sein kann.

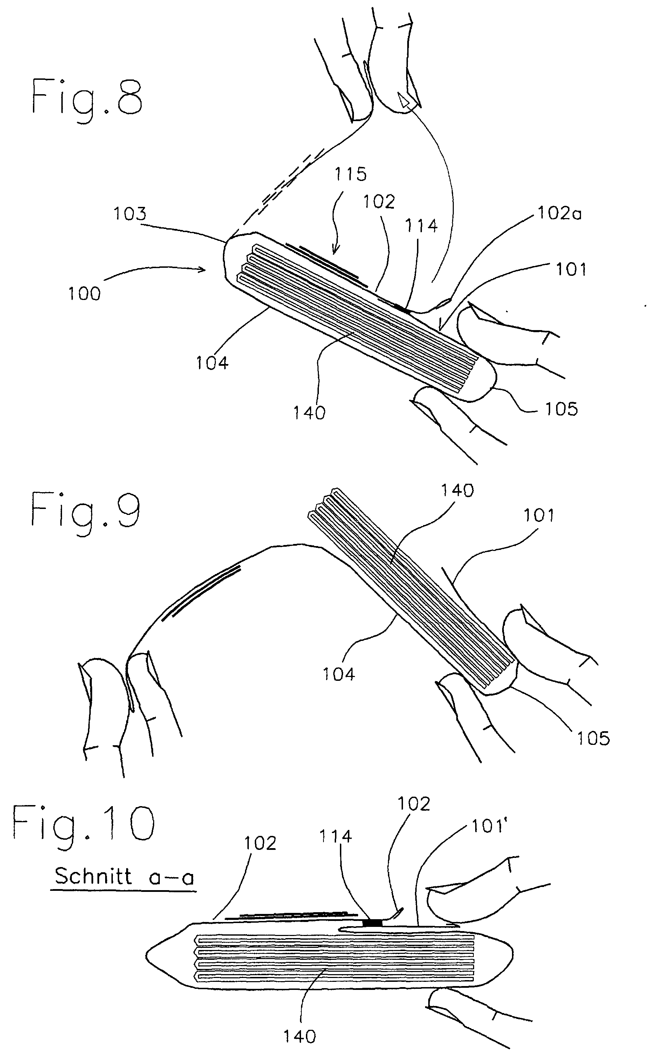

Fig. 6 zeigt eine in einer in Form einer tiefgezogenen Schale 10 ausgeführten Außenverpackung aufgenommene erfindungsgemäße Innenverpackung. Diese Innenverpackung weist eine in Form eines Schlauchbeutels 100 aus Papier gebildete Hülle auf. Diese Hülle 100 bildet eine in Fig. 6 nach oben weisende, im wesentlichen recheckige Hauptfläche 110 auf der ein Informationsträger 115 in der anhand der Fig. 1 erläuterten Weise angebracht ist. Die den Schlauchbeutel 100 bildende Materialbahn weist zwei in Längsrichtung verlaufende Ränder auf, welche sich im Bereich der Hauptfläche 110 überlappen und längs einer Verbindungslinie 114 miteinander verbunden sind. Längs ihrer quer zu der Verbindungslinie 114 bzw. den Längsrändern verlaufenden Querränder sind die einander gegenüberliegenden Abschnitte der den Schlauchbeutel 100 bildenden Materialbahn miteinander versiegelt. Wie besonders deutlich in Fig. 7 zu erkennen ist, weist der Schlauchbeutel 100 einen ersten, einen Teil der Hauptfläche 110 bildenden Abschnitt 101, einen zweiten, einen weiteren Teil der Hauptfläche bildenden Abschnitt 102, einen dritten, einen Rand des in dem Schlauchbeutel aufgenommenen Sterilgutes (hier einen Kompressenstapel) umlaufenden Abschnitt 103, einen vierten die der Hauptfläche entgegengesetzte Begrenzungsfläche der Innenverpackung bildenden Abschnitt 104 sowie einen fünften einen parallel zum dritten Abschnitt verlaufenden Rand umlaufenden Abschnitt 105 auf. Dabei überlappt der zweite Abschnitt 102 im Bereich der Hauptfläche 110 den ersten Abschnitt 101 und weist in einer senkrecht zur Umlaufachse des dritten Abschnitts und des fünften Abschnittes bzw. senkrecht zur Längsrichtung des Schlauchbeutels verlaufenden Richtung eine die Hälfte der Breite der Hauptfläche 110 überschreitende Breite auf. Auf diesem zweiten Abschnitt 102 ist der Informationsträger 115 angebracht.

Zur Herstellung der Verbindung des zweiten Abschnittes 102 mit dem ersten Abschnitt 101 ist auf der dem Sterilgut 140 zugewandten Innenseite des zweiten Abschnitts 102 längs der Verbindungslinie 14 eine siegelfähige Beschichtung aufgebracht. Dabei erstreckt sich die Verbindungslinie 114 nur über einen zentralen Teil der gesamten Länge des Schlauchbeuteils 100. Auf diese Weise wird eine Kreuzung der Verbindungslinie 114 mit den Verbindungsbereichen 120 und 130 vermieden. Die Verbindung der einzelnen Abschnitte des Schlauchbeutels im Bereich der Verbindungslinie 114 und der Verbindungslinie 120 und 130 ist so beschaffen, daß die Haltekraft dieser Längs- bzw. Querversiegelung so gering ist, daß eine Lösung der einzelnen Abschnitte voneinander ohne Papierablösung erfolgen kann. Durch die Ausbildung des zweiten Abschnitts 102 mit einer die Hälfte der Breite der Hauptfläche überschreitenden Breite wird die in Fig. 6 und 7 dargestellte dezentrale Positionierung des Informationsträgers auf der Hauptfläche ermöglicht, wobei dieser Informationsträger außerhalb des Überlappungsbereichs 112 des ersten Abschnittes und des zweiten Abschnittes angeordnet ist.

Wie in Fig. 7 angedeutet und besonders deutlich in den Fig. 8 und 9 erkennbar, ist der freiliegende Längsrand 102a des zweiten Abschnittes 102 auf sich selbst zurückgefaltet und bildet so eine verstärkte Anfaßlasche.

Wie anhand der Fig. 8 und 9 zu erkennen ist, wird die in den Fig. 6 und 7 dargestellte Innenverpackung geöffnet, indem das Sterilgut 140 mit einer Hand innerhalb des Schlauchbeutels 100 im Bereich des erste Abschnittes 101 und des vierten Abschnittes 104 festgehalten bzw. fixiert wird, während der zweite Abschnitt 102 mit der anderen Hand im Bereich der Anfaßlasche 102a erfaßt und von dem Sterilgut 140 abgehoben wird, um so eine Entnahmeöffnung zu bilden. Im Verlauf des Öffnungsvorganges wird der zweite Abschnitt 102 zusammen mit dem dritten Abschnitt 103 über den Rand des Sterilgutes 140 in Richtung auf den vierten Abschnitt 104 geklappt, wie in Fig. 9 dargestellt. Auf diese Weise wird eine sich ausgehend von der Hauptfläche 110 über den Rand des Sterilgutes 140 hinweg erstreckende Entnahmeöffnung für das Sterilgut bereitgestellt. Dabei wird durch die Beschaffenheit der Verbindung im Bereich der Verbindungslinie 114 und der Verbindungsbereiche 120 und 130 sichergestellt, daß ein Aufschälen (Peelen) der Verbindung ohne Beschädigung des Materials des Schlauchbeutels erfolgt.

Die in Fig. 9 dargestellte Entnahmeöffnung ermöglicht eine Überprüfung des Produktes und Kontrolle der Stückzahl ohne Berührung der Produkte (z.B. durch bereits kontaminierte Hände während der Operation).

Die in Fig. 10 dargestellte Ausführungsform unterscheidet sich im wesentliche nur dadurch von der anhand der Fig. 6 bis 9 erläuterten Ausführungsform, daß der erste Abschnitt 101' längs einer parallel zum Überlappungsbereich 112 verlaufenden Linie auf sich selbst zurückgefaltet ist und insgesamt ebenfalls eine die Hälfte der Breite der Hauptfläche 110 überschreitende Breite aufweist. Dabei wird der auf sich selbst zurückgefaltete Teil des ersten Abschnitts 101' nur teilweise von dem zweiten Abschnitt 102 überlappt, so daß durch die Zurückfaltung des ersten Abschnittes ein Bereich doppelter Materialstärke zum Festhalten des Sterilgutes 140 in der zu öffnenden bzw. bereits geöffneten Innenverpackung bereitgestellt wird. Weiter ist in Fig. 10 erkennbar, daß der freiliegende Rand des ersten Abschnitts 101' ähnlich wie der freiliegende Rand 102a des zweiten Abschnittes 102 zur Bildung einer verstärkten Anfaßlasche auf sich selbst zurückgefaltet ist.

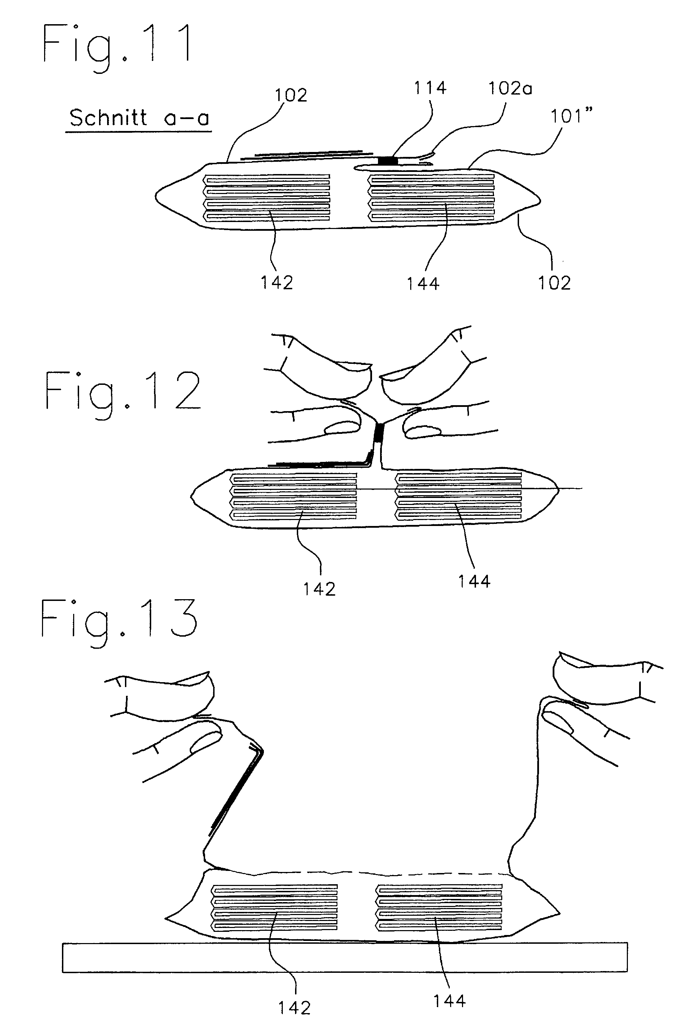

In den Fig. 11 bis 13 ist eine weitere Ausführungsform einer erfindungsgemäßen Innenverpackung dargestellt, die im besonderen zum Aufnehmen von nebeneinander angeordneten Sterilgutstapeln 142 und 144 geeignet ist. Diese Ausführungsform der Erfindung unterscheidet sich nur dadurch von der anhand der Fig. 10 erläuterten Ausführungsform, daß der erste Abschnitt 101" des Schlauchbeutels 100 derart auf sich zurückgefaltet ist, daß er vollständig von dem zweiten Abschnitt 102 überlappt wird. Zum Öffnen der Innenverpackung wird der auf sich selbst zurückgefaltete Teil des ersten Abschnittes 101" zusammen mit dem diesen überlappenden Teil des zweiten Abschnitts 102 nach oben geklappt, wie in Fig. 12 dargestellt. Dann werden die freiliegenden Ränder des ersten Abschnittes 101" und des zweiten Abschnittes 102 mit jeweils einer Hand ergriffen und voneinander getrennt. Auf diese Weise werden die Längs- und Quernähte der Innenverpackung aufgepeelt. Durch die dann entstehende jeweils feste, halbseitige Siegelnaht entsteht eine Tasche, in der das Produkt plaziert bleibt und dargereicht werden kann. Bei allen anhand der Fig. 6 bis 13 erläuterten Ausführungsformen der Erfindung kann durch eine teilweise oder unterschiedlich feste Versiegelung der Quernaht (z.B. 50%ige halbeseitige Versiegelung) der Öffnungs- bzw. Peelvorgang erleichtert werden.

Zur Durchführung der Versiegelung wird das den Schlauchbeutel bildende Papier auf seiner Innenseite mit einer siegelfähigen Beschichtung versehen. Dabei ist die Haltekraft der Längs- und Quersielung jedoch nur so fest, daß sie ohne Papierablösung gelöst werden kann. Wie eingangs bereits erläutert ist bei der anhand der Fig. 6 bis 9 erläuterten Ausführungsform die beschichtete Innenseite mit der unbeschichteten Außenseite der Verpackung verbunden. Bei den anhand der Fig. 10 bis 13 erläuterten Ausführungsformen werden beide Innenseiten beschichtet und miteinander versiegelt.

Als Material für die Innenverpackung können je nach Sterisisationsverfahren teilbeschichtete (Rahmen-) oder vollflächig beschichtete Papiere oder Kunststoffe sowie Verbundpackstoffe in unterschiedlichen Zusammensetzungen, kalt- oder heißsiegelfähig, verwendet werden. Vollbeschichtete Papiere werden z.B. mit γ-Strahlen sterilisiert. Für EO oder Dampfsterisisation sind rahmenbeschichtete Papiere erforderlich.

## Patentansprüche

1. Innenverpackung für eine Packung zum Lagern von Sterilgut mit einer das Sterilgut (140, 142, 144) aufnehmenden, mindestens eine vorzugsweise im wesentlichen ebene Hauptfläche (110) aufweisenden und zum Bilden einer Entnahmeöffnung für das Sterilgut vorbereiteten Hülle (100), bei der die Hülle (100) zum Bilden der Entnahmeöffnung im Bereich der Hauptfläche (110) vorbereitet ist, die Hülle (100) eine das Sterilgut zumindest teilweise umlaufende Materialbahn aufweist und die Materialbahn einen ersten Abschnitt (101) und einen zweiten Abschnitt (102) aufweist, von denen jeder einen Teil der Hauptfläche (110) bildet, wobei sich diese Abschnitte (101, 102) im Bereich der Hauptfläche (110) zumindest teilweise überlappen**dadurch gekennzeichnet, daß** ein freiliegender Rand (102a) des ersten Abschnittes (101) und/oder des zweiten Abschnittes (102) zur Bildung einer verstärkten Anfaßlasche zumindest teilweise auf sich selbst zurückgefaltet ist.

2. Innenverpackung nach Anspruch 1, **dadurch gekennzeichnet, daß** die Hülle (100) zum Bilden einer sich ausgehend von der Hauptfläche (110) bis über einen daran angrenzenden Rand des darin aufgenommenen Sterilgutes (140, 142, 144) hinweg erstreckenden Entnahmeöffnung vorbereitet ist.

3. Innenverpackung nach Anspruch 1 oder 2, **dadurch gekennzeichnet, daß** die Materialbahn einen ersten, einen Teil der Hauptfläche (110) bildenden Abschnitt (101), einen zweiten, einen weiteren Teil der Hauptfläche (110) bildenden Abschnitt (102), einen dritten, eine erste Umlaufachse teilweise umlaufenden Abschnitt (103), einen vierten, die der Hauptfläche (110) entgegengesetzte Begrenzungsfläche der Innenverpackung bildenden Abschnitt (104) und einen fünften, eine zweite, vorzugsweise etwa parallel zu der ersten Umlaufachse verlaufende Umlaufachse teilweise umlaufenden Abschnitt (105) aufweist.

4. Innenverpackung nach einem der Ansprüche 1 bis 3, **dadurch gekennzeichnet, daß** sich der erste Abschnitt (101) und der zweite Abschnitt (102) längs etwa parallel zu den Umlaufachsen verlaufenden Überlappungsbereich (112) überlappen.

5. Innenverpackung nach einem der Ansprüche 1 bis 4, **dadurch gekennzeichnet, daß** der erste Abschnitt (101) und der zweite Abschnitt (102) in dem Überlappungsbereich (112) lösbar miteinander verbunden, insbesondere verklebt oder versiegelt sind.

6. Innenverpackung nach Anspruch 5, **dadurch gekennzeichnet, daß** die Haltekraft der Verbindung so gering ist, daß beim Lösen der Verbindung keine Beschädigung des ersten Abschnittes (101) oder zweiten Abschnittes (102) erfolgt.

7. Innenverpackung nach einem der Ansprüche 3 bis 6, **dadurch gekennzeichnet, daß** der zweite Abschnitt (102) auf seiner dem Sterilgut (140, 142, 144) zugewandten Seite mit dem ersten Abschnitt (101) verbunden ist und in einer sich senkrecht zu den Umlaufachsen erstreckende Richtung eine die Hälfte der Breite der Hauptfläche (110) überschreitende Breite aufweist.

8. Innenverpackung nach einem der Ansprüche 1 bis 7, **dadurch gekennzeichnet, daß** der erste Abschnitt (101) längs einer parallel zum Überlappungsbereich (112) verlaufenden Linie auf sich selbst zurückgefaltet ist und vorzugsweise in einer sich senkrecht zu den Umlaufachsen verlaufenden Richtung eine die Hälfte der Breite der Hauptfläche (110) überschreitende Breite aufweist.

9. Innenverpackung nach einem der Ansprüche 3 bis 8, **dadurch gekennzeichnet, daß** der erste Abschnitt (101) und/oder der zweite Abschnitt (102) längs mindestens eines quer zu den Umlaufachsen verlaufenden Verbindungsbereiches (120, 103) mit dem vierten Abschnitt (104) verbunden, insbesondere verklebt oder versiegelt ist, wobei die Haltekraft dieser Verbindung vorzugsweise so gering ist, daß beim Lösen der Verbindung keine Beschädigung des ersten, zweiten oder vierten Abschnittes (101, 102, 104) erfolgt.

10. Innenverpackung nach Anspruch 9, **dadurch gekennzeichnet, daß** sich die Verbindung des ersten Abschnittes (101) mit dem zweiten Abschnitt (102) nicht mit der Verbindung des ersten bzw. zweiten Abschnittes (101, 102) mit dem vierten Abschnitt (104) kreuzt.

## Claims

1. Inner packing for a package for a sterile product with a wrapping (100) prepared to form the opening for removing the sterile product with at least one preferably basically flat main surface (110) receiving the sterile product (140, 142, 144), in which the wrapping (100) is prepared to form the opening for removing in the area of the main surface (110), the wrapping (100) has a length of material at least partly running round the sterile product and the length of material has a first section (101) and a second section (102), each of which forms a part of the main surface (110), in which these sections (101, 102) at least partly overlap each other in the area of the main surface (110), **characterised by** the fact that a free edge (102a) of the first section (101) and/or the second section (102) is folded back on itself at least partly to form a reinforced holding loop.

2. Inner packing according to claim 1, **characterised by** the fact that the wrapping (100) is prepared to form an opening for removing which extends from the main surface (110) over an adjacent edge of the sterile product received by it (140, 142 144).

3. Inner packing according to claim 1 or 2, **characterised by** the fact that the length of material has a first section (101) forming a part of the main surface (110), a second section (102) forming a further part of the main surface (110), a third section (103) partly running round a first rotational axis, a fourth section (104) forming the boundary surface of the inner packing opposite the main surface (110) and a fifth section (105) partly running round a second rotational axis preferably running parallel to the first rotational axis.

4. Inner packing according to one of claims 1 to 3, **characterised by** the fact that the first section (101) and the second section (102) overlap each other along an overlapping area (112) running approximately parallel to the rotational axes.

5. Inner packing according to one of claims 1 to 4, **characterised by** the fact that the first section (101) and the second section (102) are detachably connected to each other in the overlapping area (112), particularly stuck or sealed together.

6. Inner packing according to claim 5, **characterised by** the fact that the holding force of the connection is so small that when the connection is released there is no damage to the first section (101) or the second section (102).

7. Inner packing according to one of claims 3 to 6, **characterised by** the fact that the second section (102) is connected to the first section (101) on the side facing the sterile product (140, 142, 144) and has a width exceeding half the width of the main surface (110) in a vertical direction to the rotational axes.

8. Inner packing according to one of claims 1 to 7, **characterised by** the fact that the first section (101) is folded back on itself along a line running parallel to the overlapping area (112) and preferably has a width exceeding half the width of the main surface (110) in a vertical direction to the rotational axes.

9. Inner packing according to one of claims 3 to 8, **characterised by** the fact that the first section (101) and/or the second section (102) is connected to the fourth section (104) along at least one connecting area (120, 103) running crosswise to the rotational axes, particularly stuck or sealed together, in which the holding force of this connection is preferably so small that when the connection is released there is no damage to the first, second or fourth section (101, 102, 104).

10. Inner packing according to claim 9, **characterised by** the fact that the connection of the first section (101) to the second section (102) does not cross with the connection of the first or second section (101, 102) to the fourth section (104).

## Revendications

1. Emballage intérieur pour un emballage pour le stockage d'un produit stérile avec une enveloppe (100) recevant le produit stérile (140, 142, 144), présentant au moins une face principale (110) de préférence sensiblement plane et préparée pour la formation d'une ouverture de retrait du produit stérile, où l'enveloppe (100) est préparée pour former l'ouverture de retrait dans la zone de la face principale (110), l'enveloppe (100) présente une bande de matériau entourant le produit stérile au moins partiellement, et la bande de matériau présente un premier tronçon (101) et un second tronçon (102) dont chacun constitue une partie de la face principale (110), où ces tronçons (101, 102) se recouvrent dans la zone de la face principale (110) au moins partiellement, **caractérisé en ce qu'**un bord libre (102a) du premier tronçon (101) et/ou du deuxième tronçon (102), pour former une languette de préhension renforcée, est au moins partiellement replié sur lui-même.

2. Emballage intérieur selon la revendication 1, **caractérisé en ce que** l'enveloppe (100) est préparée pour la formation d'une ouverture de retrait s'étendant depuis la face principale (110) jusqu'au-delà d'un bord avoisinant celle-ci du produit stérile (140, 142, 144) reçu dans celle-ci.

3. Emballage intérieur selon la revendication 1 ou 2, **caractérisé en ce que** la bande de matériau présente un premier tronçon (101) formant une partie de la face principale (110), un deuxième tronçon (102) formant une autre partie de la face principale (110), un troisième tronçon (103) s'étendant partiellement autour d'un premier axe tournant, un quatrième tronçon (104) formant la face de délimitation de l'emballage intérieur opposée à la face principale (110) et un cinquième tronçon (105) s'étendant partiellement autour d'un deuxième axe tournant s'étendant de préférence parallèlement au premier axe tournant.

4. Emballage intérieur selon l'une des revendications 1 à 3, **caractérisé en ce que** le premier tronçon (101) et le deuxième tronçon (102) se chevauchent le long d'une zone de chevauchement (112) s'étendant à peu près parallèlement aux axes tournants.

5. Emballage intérieur selon l'une des revendications 1 à 4, **caractérisé en ce que** le premier tronçon (101) et le deuxième tronçon (102) sont reliés relâchablement l'un à l'autre dans la zone de chevauchement (112), en particulier en étant collés ou scellés.

6. Emballage intérieur selon la revendication 5, **caractérisé en ce que** la force de retenue de l'assemblage est tellement faible que lors du relâchement de l'assemblage, ni le premier tronçon (101) ni le deuxième tronçon (102) n'est endommagé.

7. Emballage intérieur selon l'une des revendications 3 à 6, **caractérisé en ce que** le deuxième tronçon (102) est relié à son côté orienté vers le produit stérile (140, 142, 144) au premier tronçon (101) et présente, dans une direction s'étendant perpendiculairement aux axes tournants, une largeur dépassant la moitié de la largeur de la face principale (110).

8. Emballage intérieur selon l'une des revendications 1 à 7, **caractérisé en ce que** le premier tronçon (101) est replié sur lui-même le long d'une ligne s'étendant parallèlement à la zone de recouvrement (112) et présente de préférence, dans une direction s'étendant perpendiculairement aux axes tournants, une largeur dépassant la moitié de la largeur de la face principale (110).

9. Emballage intérieur selon l'une des revendications 3 à 8, **caractérisé en ce que** le premier tronçon (101) et/ou le deuxième tronçon (102), le long d'au moins une zone de liaison (120, 103) s'étendant transversalement aux axes tournants, est relié au quatrième tronçon (104), en particulier est collé ou scellé, où la force de retenue de cette liaison est de préférence tellement faible que lors du relâchement de la liaison, aucun parmi le premier, deuxième ou quatrième tronçon (101, 102, 104) soit endommagé.

10. Emballage intérieur selon la revendication 9, **caractérisé en ce que** la liaison du premier tronçon (101) avec le deuxième tronçon (102) ne se croise pas avec la liaison du premier respectivement deuxième tronçon (101, 102) avec le quatrième tronçon (104).
